# EUROPEAN PATENT SPECIFICATION

(11) **EP 4 613 183 B1**
(45) Date of publication and mention of the grant of the patent: **23.09.2026**
(21) Application number: 25160552.3
(22) Date of filing: 27.02.2025
(51) Int. Cl.: A61B 5/0205, A61B 5/024, A61B 5/16

(54) **METHOD AND SYSTEM FOR MONITORING ATTAINMENT OF FLOW STATE FOR ACTIVITY BY USER**
VERFAHREN UND SYSTEM ZUR ÜBERWACHUNG DES ERREICHENS EINES FLUSSZUSTANDS FÜR AKTIVITÄT DURCH EINEN BENUTZER
PROCÉDÉ ET SYSTÈME DE SURVEILLANCE DE L'ACQUISITION DE L'ÉTAT DE FLUX POUR UNE ACTIVITÉ PAR UN UTILISATEUR

(30) Priority: 05.03.2024 FI 20245274
(43) Date of publication of application: 10.09.2025
(73) Proprietor: Pixieray Oy, 02630 Espoo (FI)
(72) Inventor: Melakari, Rebecca, 02140 Espoo (FI); Lehmuskallio, Harri, 00100 Helsinki (FI)
(74) Representative: Moosedog Oy

(56) References cited:
- US-A1- 2016 196 758
- US-A1- 2020 012 403
- US-A1- 2022 071 535
- US-A1- 2022 240 824

## Description

### TECHNICAL FIELD

The present disclosure relates to methods for monitoring attainment of flow state for activities by users. Moreover, the present disclosure relates to systems for monitoring attainment of flow state for activities by users.

### BACKGROUND

There is a growing intersection of technology, well-being, and user experience, which aims to provide a system that monitors and enhances an individual's experience of achieving a state of flow while being involved in various activities. Conventionally, fitness trackers are used for monitoring the individual using biometric measurements, such as monitoring heart beats, age, weight, body posture, and the like, which are indicative of an overall physical well-being of the individual.

However, existing methods utilized for attaining the performance of user are associated with several limitations. Herein, the existing techniques use biometric measurements to determine performance of the individual, wherein the biometric measurements are collected by particularly focusing on the individual's physical activities and sleep activity. Such biometric measurements lead to an incomplete understanding of factors which influencing an overall performance. Conventionally, sleep trackers are used to calculate readiness scores from previous scores, sleep activity and a quality of sleep of the individual. Herein, the readiness score provides a guidance as to how to approach the new day, based only on the sleep on the individual. This can be limiting, as other factors affecting the overall well- being of the individual is not taken into consideration. Furthermore, the sleep trackers do not provide feedback in real time or in near-real time for the individual to make appropriate adjustments to enhance the well-being. Moreover, conventional fitness trackers are used to calculate a strain score for guiding the individual towards a physical performance which promotes the overall well-being of the individual. The strain score primarily focuses on physical exertion of the individual, thereby providing an overall measure of stress on the individual. However, this can be limiting, as other factors affecting the overall well-being of the individual is not taken into consideration.

US 2022/071535 A1 discloses a method and system to determine the mental state of a user when performing a task.

Therefore, in light of the foregoing discussion, there exists a need to overcome the aforementioned drawbacks associated with existing techniques for monitoring attainment of flow state for users' activity.

### SUM MARY

The aim of the present disclosure is to provide methods and systems for monitoring attainment of flow state for activities by users, to determine a level of engagement of the user in a dynamic manner. The aim of the present disclosure is achieved by methods and systems for monitoring attainment of flow state for activities by users as defined in the appended independent claims to which reference is made to. Advantageous features are set out in the appended dependent claims.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

FIG. 1 illustrates steps of a method for monitoring attainment of a flow state for an activity by a user, in accordance with an embodiment of the present disclosure;
FIG. 2 is an architecture of a system for monitoring attainment of a flow state for an activity by a user, in accordance with an embodiment of the present disclosure; and
FIGs. 3A, 3B, and 3C illustrate various views of an interactive user interface, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides a method for monitoring attainment of a flow state for an activity by a user, the method comprising:
receiving a first sensor data from an eye-tracking means, and a second sensor data from a heart rate monitoring device, wherein the first sensor data and the second sensor data are collected while the user is engaged in the activity;
determining an eye fixation frequency and an eye fixation percentage by processing the first sensor data, and determining a heart rate and a heart rate variability by processing the second sensor data;
determining a focus index of the user, based on the eye fixation frequency and the eye fixation percentage;
determining a stress index of the user, based on the heart rate and the heart rate variability;
determining a flow index of the user, based on a difference between the focus index and the stress index;
detecting that the user has attained the flow state for the activity, when the determined flow index is equal to or greater than a predefined flow index for the activity;
receiving a third sensor data from a tracking means; and
determining at least one of: a pose of the user's head, a pose of a head-mounted device worn by the user on the user's head, a pose of a user-interaction controller that is held or worn by the user, by processing the third sensor data,
wherein the at least one of: the pose of the user's head, the pose of the head-mounted device worn by the user on the user's head, the pose of the wearable device worn by the user, is utilized when determining at least one of: the focus index, the stress index, a fatigue index.

In a second aspect, the present disclosure provides a system for monitoring attainment of a flow state for an activity by a user, the system comprising:
an eye-tracking means;
a heart rate monitoring device;
a tracking means; and
at least one processor configured to:
   receive a first sensor data from the eye-tracking means, and a second sensor data from the heart rate monitoring device, wherein the first sensor data and the second sensor data are collected while the user is engaged in the activity;
   determine an eye fixation frequency and an eye fixation percentage by processing the first sensor data, and determining a heart rate and a heart rate variability by processing the second sensor data;
   determine a focus index of the user, based on the eye fixation frequency and the eye fixation percentage;
   determine a stress index of the user, based on the heart rate and the heart rate variability;
   determine a flow index of the user, based on a difference between the focus index and the stress index;
   detect that the user has attained the flow state for the activity, when the determined flow index is equal to or greater than a predefined flow index for the activity;
   receive a third sensor data from the tracking means; and
   determine at least one of: a pose of the user's head, a pose of a head-mounted device worn by the user on the user's head, a pose of a user-interaction controller that is held or worn by the user, by processing the third sensor data,
   wherein the at least one of: the pose of the user's head, the pose of the head-mounted device worn by the user on the user's head, the pose of the wearable device worn by the user, is utilized when determining at least one of: the focus index, the stress index, a fatigue index.

The present disclosure provides the aforementioned first aspect and the aforementioned second aspect, wherein the attainment of the flow state of the user is determined dynamically, when the user is engaged in the activity. In this regard, the first sensor data and the second sensor data are processed to determine the user's physiological state and cognitive state during the activity. This is used to determine whether the user has attained the flow state or not. In this regard, a level of user's engagement is monitored in real time or near-real time, wherein such level of the user's engagement can be determined by monitoring features of the user's body, for example such as eyelids of each eye, pupils of each eye, heartbeats per minute, and similar. Hence, determining the focus index and the stress index is used to determine whether the user has attained the slow state for the activity. Moreover, the third sensor data is processed to determine at least one pose of the user. Hence, by determining at least one of: the focus index, the stress index, the fatigue index, precise information is provided dynamically about the user's movements, orientation or position, thus enhancing user's experience. In this way, the method and the system facilitate a user-friendly, simple and effective technique for monitoring attainment of the flow state for the activity.

Throughout the present disclosure, the term *"flow state"* refers to a state of immersion while being in focus, involvement, and enjoyment in a process of the activity which is performed by the user. In other words, when the user attains the flow state for the activity, it means that the user is fully immersed while being in energized focused, full involvement, and enjoyment while being engaged in the activity. It will be appreciated that flow states for different activities can be different, as such different activities require different levels of focus, different levels of involvement, and different levels of enjoyment of the user. The flow state of the user is accurately monitored when the user is engaged in the activity, to determine when the flow state has been attained. This allows the user to determine how to attain the flow state with respect to a particular activity, and thus utilize their full potential for the particular activity.

Throughout the present disclosure, the term *"eye-tracking means"* refers to a specialized equipment that is employed to detect at least an extent of closure of the user's eyes, and/or a movement of the user's eyes. Such eye-tracking is performed when a system comprising the eye-tracking means, in operation, is worn by the user. Optionally, the eye-tracking means is further employed to detect and/or follow a gaze direction of the user of the system. Optionally, the eye-tracking means is implemented by way of at least one of: contact lenses having sensors, cameras monitoring features of the eyes, sensors arranged on a frame of the system. Such features may comprise at least one of: a shape of a pupil of each eye, a size of the pupil, corneal reflections of light emanating from a real-world environment from a surface of each eye, a relative position of the pupil with respect to the corneal reflections, a relative position of the pupil with respect to corners of each eye. Such eye-tracking means are well-known in the art. The eye-tracking means is configured to collect the first sensor data and send the first sensor data to at least one processor (as described later). It will be appreciated that the first sensor data is collected repeatedly by the eye-tracking means throughout an operation of the system, as the extent of closure and/or the movement of each eye of the user keeps changing whilst gaze of the user's eyes keeps changing. An up-to-date first sensor data allows for determining the flow state for the activity of the user. In an instance, when the eye-tracking means is implemented as the camera, the first sensor data is in form of images of the user's eyes. In another instance, when the eye-tracking means is implemented by way of contact lenses having sensors, the first sensor data is data collected from the sensors.

The term *"heart rate monitoring device"* refers to a specialized device that is designed to measure and track the user's heart rate, optionally, in real time. Such measuring and tracking of the user's heart rate is performed when the system comprising the heart rate monitoring device, in operation, is worn by the user. Optionally, the heart rate monitoring device is implemented by way of at least one of: an optical sensor, an electrocardiograph sensor, photoplethysmography (PPG) sensor. Such heart rate monitoring devices are well-known in the art. The heart rate monitoring device is configured to collect the second sensor data and send the second sensor data to the at least one processor. It will be appreciated that the second sensor data is collected repeatedly by the heart rate monitoring device throughout the operation of the system, as the heart rate of the user changes based on the activity of the user. An up-to-date second sensor data allow for determining the flow state for the activity of the user. In an instance, when the heart rate monitoring device is implemented as the optical sensor, the second sensor data is in form of amount of light intensity caused by variations in blood volume with each heartbeat. In another instance, when the heart rate monitoring device is implemented by electrocardiograph sensor, the second sensor data is electrical data related to heart activity of the user.

A technical effect of collecting the first sensor data and the second sensor data is that it enables to determine a visual attention and physiological responses of the user during the particular activity. This is beneficial in determining whether the user is actively involved or not.

It will be appreciated that the at least one processor is communicably coupled with the eye-tracking means, and the heart rate monitoring device. The at least one processor could be implemented as any one of: a microprocessor, a microcontroller, or a controller. As an example, the at least one processor could be implemented as an application-specific integrated circuit (ASIC) chip, or a reduced instruction set computer (RISC) chip.

Throughout the present disclosure, the term *"eye fixation frequency"* refers to a number of times the user's eyes fixate on a particular region within a duration of time. The term *"eye fixation percentage"* refers to the proportion of time spent in fixating on the particular region when compared to a total duration of observation time by the user's eyes. The first sensor data is used to determine the eye fixation frequency, by processing the first sensor data to determine the frequency and/or pattern of changes in at least one of: the corneal reflections of light emanating from the real-world environment from the surface of each eye, the relative position of the pupil with respect to the corneal reflections, the relative position of the pupil with respect to corners of each eye. In this regard, a given instance where the gaze of the user's eyes remains relatively stable within the particular region for the duration of time is determined to be the eye fixation. Furthermore, the first sensor data is processed to determine a total time spent on the eye fixations, and dividing said total time spent on the eye fixations by the total duration of observation time, to determine the eye fixation percentage. Herein, the first sensor data is processed by employing an image processing algorithm to analyse at least one of: the corneal reflections of light emanating from the real-world environment from the surface of each eye, the relative position of the pupil with respect to the corneal reflections, the relative position of the pupil with respect to corners of each eye. Such image processing algorithms are well-known in the art.

The second sensor data is used to determine the heart rate, by processing the second sensor data to determine time intervals between successive heartbeats of the user, by providing information regarding the electrical signals or changes in the blood volume related to each heartbeat. Optionally, heart rate processing algorithms are used to analyse the second sensor data to detect peaks and patterns corresponding to the heart beat of the user, thus allowing determination of the heart rate. Such processing algorithms are well-known in the art. Herein, the term *"heart rate"* refers to the number of heart beats per minute (bpm) of the user when she/he is performing the particular activity. The heart beat can vary depending on factors, for example, such as physical activity, emotional state, overall health, and similar. The term *"heart rate variability"* refers to a variation in the time intervals between the successive heartbeats of the user.

Throughout the present disclosure, the term *"focus index"* refers to a level of focus (namely, a level of concentration) of the user when she/he is engaged in the activity. Optionally, the focus index emulates a state machine, wherein the state machine receives the eye fixation frequency and the eye fixation percentage and provides a focus score, wherein the focus score is indicative of consistency of the eye fixation frequency and the eye fixation percentage on the given region. The focus score is then used to determine the focus index. Optionally, the focus index is directly related to the eye fixation frequency. Similarly, optionally, the focus index is directly related to the eye fixation percentage. Moreover, using the eye fixation frequency and the eye fixation percentage to determine the focus index is beneficial in providing an accurate understanding of the user's visual attention, thereby leading to a better performance by the user in the activity. Herein, the eye fixation frequency and the eye fixation percentage are specific, measurable parameters, wherein the eye fixation frequency is calculated from pattern of changes in at least one of: the corneal reflections of light emanating from the real-world environment from the surface of each eye, the relative position of the pupil with respect to the corneal reflections, the relative position of the pupil with respect to corners of each eye, and the eye fixation percentage, and the eye fixation percentage is calculated by dividing total time spent on the eye fixations by the total duration of observation time. Hence, the focus index is derived from specific measurable parameters.

Optionally, the focus index depends on the activity the user is engaged in. Optionally, the focus index at least lies in a range from 0 to 1, 0 to 10, 0 to 25, 0 to 100, and similar. In a first exemplary use case scenario, the activity in which the user may be engaged in may comprise at least one of: making notes, writing emails. The user may have a high eye fixation frequency and a high eye fixation percentage. Hence, the focus index may lie in a range of 0.76 to 1, thereby indicating that the user may have deep and active focus in the activity. In a second exemplary use case scenario, the activity in which the user may be engaged in may comprise scrolling social media. The user may have a high eye fixation frequency and low eye fixation percentage. Hence, the focus index may lie in a range of 0.51 to 0.75, thereby indicating that the user may have divided/scanning focus. In a third exemplary use case scenario, the activity in which the user may be engaged in may comprise at least one of: watching a movie, listening to music, day dreaming. The user may have a low eye fixation frequency and a high eye fixation percentage. Hence, the focus index may lie in a range of 0.26 to 0.50, thereby indicating that the user may have static focus. In a fourth exemplary use case scenario, the user may be engaged in such activity from which the user may be continuously distracted. The user may have a low eye fixation frequency and a low eye fixation percentage, thereby indicating that the user's attention may be completely divided.

Optionally, the range of focus index varies as per a performance of the user while being engaged in the activity. In a first example, the focus index may lie in a range of 0 to 50. Herein, when the focus index is 20 out of 50, it may indicate that the user has moderate focus in the activity. Alternatively, when the focus index is 10 out of 50, it may indicate that the user has low focus in the activity, which means that the user is likely to be distracted or not actively engaged from/in the activity. In a second example, the focus index may lie in a range of 0 to 90. Herein, when the focus index is 80 out of 90, it may indicate that the user may be in deep focus, which means that the user may fully involved in the activity. Alternatively, when the focus index is 45 out of 90, it may indicate that the user has moderate focus in the activity.

Throughout the present disclosure, the term *"stress index"* refers to a level of stress experienced by the user while performing the activity. Optionally, the stress index is directly related to the heart rate. Herein, when the heart rate is higher than a normal range of heart rate (i.e., the normal range of heart rate typically lies between 60 to 100 bpm for a healthy adult in a resting condition), a score of the stress index is high, indicating that the user is stressed while being engaged in the activity.

Alternatively, when the heart rate is lower than the normal range of heart rate, the score of the stress index is low, indicating that the user is less stressed while being engaged in the activity. Optionally, the stress index is inversely related to the heart rate variability. Herein, a high heart rate variability indicates better resilience, and adaptability of the user's body to transition between states of relaxation and excitement efficiently. Alternatively, a low heart rate variability indicates that the user is fatigued, and the user's body is not able to transition between states of relaxation and excitement efficiently. Moreover, using the heart rate and the heart rate variability to determine the stress index is beneficial in providing an accurate understanding of physiological response of the user to stressors, thereby leading to a better performance by the user in the activity. Hence, the stress index is derived from concrete physiological measurement, i.e., heart rate and heart rate variability.

Optionally, the stress index at least lies in a range from 0 to 1, 0 to 10, 0 to 25, 0 to 100, and similar. The stress index depends on the activity the user is engaged in. Continuing in reference with the second exemplary use case scenario, the stress index may lie in the range from 0 to 1. The heart rate of the user may be within the normal range of heart rate and the heart rate variability is high. Herein, the stress index may be 0.5, thereby indicating that the stress may be low. Continuing in reference with the fourth exemplary use case scenario, the stress index may lie in the range from 0 to 1. The heart rate of the user may be higher than the normal range of heart rate and the heart rate variability is low. Herein, the stress index may be 0.9, thereby indicating that the stress experienced by the user may be high. Optionally, the stress index provides information regarding a level of relaxation experienced by the user. Herein, the level of relaxation is a state of the stress index being low (i.e., below a predefined value).

Throughout the present disclosure, the *"flow index"* is a numerical indicator of the flow state of the user, which is used to determine whether the user has attained the flow state of the activity or not. The flow index is determined using a following formula (1), $Flow Index = Focus Index - Stress Index$

Herein, an increase in the focus index has a positive impact on the flow index by enhancing a likelihood of entering or maintaining the flow state of the user. Conversely, an increase in the stress index has a negative impact on the flow index by potentially impeding the attainment of the flow state due to distractions or reduced engagements. Hence, the focus index and the stress index are combined to determine a balance between high focus and low stress, which is conducive to attaining the flow state. Therefore, this facilitates the user to adjust a level of difficulty or complexity of the activity. A technical effect obtained by determining the difference between the focus index and the stress index is that the flow state is determined in an alternative manner, as determining the flow state by itself is not possible to be determined exactly. Beneficially, as the first sensor data and the second sensor data are measured in a precise manner, it enables immediate creation of the flow index even with a minimum amount of data. Moreover, the flow index is not an arbitrary value, but rather a calculated parameter based on scientifically measurable inputs with clear physiological significance.

The term *"predefined flow index"* refers to a threshold value for the user engaged in the activity, wherein the threshold value is determined based on empirical data or prior observations specific to the activity that the user is engaged in. Hence, when the determined flow index is equal to or greater than the predefined flow index for the activity, it means that there is the likelihood that the user has attained the flow state for the activity. In other words, when the determined flow index is equal to or greater than the predefined flow index for the activity, the user is fully focused and involved in said activity.

Optionally, the predefined flow index is different for different activities. The predefined flow index for an activity could be defined more specifically, and lies within the range of the flow index. Herein, a value of the predefined flow index depends on the range of the flow index. For example, the predefined threshold could lie in a range of 0.2 to 0.8 when the flow index lies in the range of 0 to 1, the predefined threshold could lie in a range of 1 to 20 when the flow index lies in the range of 0 to 21.

Hence, monitoring the flow index facilitates to determine the user's level of stress, level of focus, level of engagement, and similar, related to the activity associated with the flow state for the activity. In this regard, the likelihood of the user to attain the flow state characterized by a sense of satisfaction, fulfilment, deep focus, heightened engagement during the activity is determined.

Optionally, the method further comprises detecting that the user has not attained the flow state for the activity, when the determined flow index is less than the predefined flow index for the activity. Hence, when the determined flow index of the user is lesser than the predefined flow index of the activity, it means that the likelihood of that the user attaining the flow state for the activity has decreased. This means that the user is not focused and/or involved in said activity.

Throughout the present disclosure the term *"tracking means"* refers to an equipment that is used to measure and record the third sensor data, wherein the third sensor data is indicative of at least one of: a motion, an orientation, a position of an object in a three-dimensional (3D) space. Optionally, the tracking means is implemented as an inertial measurement unit (IMU), wherein the IMU comprises at least one of: an accelerometer, a gyroscopes, a magnetometer. It will be appreciated that the third sensor data is collected repeatedly by the tracking means throughout an operation of the system, as at least one of: the motion, the orientation, the position of the object in the 3D spaces keeps changing. An up-to-date third sensor data allows for determining a relative position of the object. In an instance, when the tracking means is implemented as the accelerometer, the third sensor data comprises measurements related to linear acceleration along multiple axes. In another instance, when the tracking means is implemented as the gyroscope, the third sensor data comprises measurements related to angular velocity or changes in orientation along different axes. In yet another instance, when the tracking means is implemented as the magnetometer, the third sensor data comprises measurements related to strength and direction of the magnetic field around the tracking means.

The tracking means could be implemented as an internal component to the system, as a tracking system external to the system, or a combination thereof. Optionally, at least one data processing algorithm is employed to process the third sensor data. the third sensor data may be in form of images, Inertial Measurement Unit (IMU) values, motion sensor data values, magnetic field strength values, or similar. Correspondingly, requisite data processing algorithm(s) is/are employed to process the third sensor data, to track at least one of: the pose of the user's head, the pose of the head-mounted device worn by the user on the user's head, the pose of the user-interaction controller that is held or worn by the user. Herein, the term *"pose"* encompasses both position and orientation. Examples of the at least one data processing algorithm include a feature detection algorithm, an environment mapping algorithm, a pose data extrapolation algorithm, and the like.

Herein, the term *"head-mounted device"* refers to a specialized equipment that is configured to present an extended-reality (XR) environment to the user when said head-mounted device (HMD), in operation, is worn by the user on his/her head. The HMD is implemented, for example, as an XR headset, a pair of XR glasses, and the like, that is operable to display a visual scene of the XR environment to the user. The term *"extended-reality"* encompasses virtual reality (VR), augmented reality (AR), mixed reality (MR), and the like. The term "head-mounted device" can also refer to pair of glasses, such as eye glasses, goggles, smart glasses or a like.

Optionally, the HMD is comprised in an optical apparatus. The term *"optical apparatus"* refers to an apparatus that is to be worn over the eyes of the user. Examples of the optical apparatus include, but are not limited to, a pair of glasses, a pair of sunglasses, and smart glasses.

In an instance, in the optical apparatus, when the eye-tracking means is implemented by way of lenses having sensors, the first sensor data is data collected from the sensors. In yet another instance, in the optical apparatus, when the eye-tracking means is implemented by way of light sensors, the first sensor data comprises light signals detected by the light sensors. As an example, when the optical apparatus may comprise the pair of glasses, the eye-tracking means may be implemented by way of at least one infrared (IR) light-emitting diode (LED) and at least one IR receiver, arranged on a frame of the pair of eyeglasses. Herein, the at least one IR LEDs may emit light towards the eye, and light reflected from surface of the eye may be incident on the at least one IR receiver. This reflection may be used to determine the gaze direction of the user's eyes.

Moreover, the term *"user-interaction controller"* refers to a specialized equipment that is employed by the user to interact with the XR environment. Notably, the user interacts with the XR environment by providing input(s) to the tracking means, via the user-interaction controller. The user-interaction controller is communicably coupled with the tracking means. Herein, the coupling could be wired, wireless, or a combination thereof. Optionally, the user-interaction controller is implemented as at least one of: a keyboard, a mouse, a touchpad, a push button controller, a joystick, a gamepad, an interactive board, a tablet computer, a laptop computer, a trackball, an XR controller.

In an instance, the pose of the user's head can be used to infer attention or a level of focus. In this regard, certain poses of the user's head (for example, such as a no movement of the head due to a prolonged fixed gaze, repeated head movements), is used to deduce periods of focused attention, which contributes to determination of the focus index. For example, when the user may be reading a book or listening to an audio version of the book, the pose of the head may not change, thereby indicating a highly focused state of the user. In another instance, at least one of: the pose of the user's head, the pose of the head-mounted device worn by the user on the user's head, the pose of the user-interaction controller that is held or worn by the user, can be used to infer behaviour related to stress. In this regard, certain poses of the head or a body part which the user-interaction controller is held (for example, such as irregular head movements, irregular orientations of the body part), is used to deduce discomfort or stress, which contributes to determination of the stress index. In yet another instance, at least one of: the pose of the user's head, the pose of the head-mounted device worn by the user on the user's head, can be used to infer changes in posture or head position, through which fatigue can manifest. In this regard, changes in pose of the head are used to identify patterns associated with fatigue, which contributes to determination of the fatigue index. In this regard, the term *"fatigue index"* refers to a metric that is used to measure a level of fatigue experienced by the user when engaged in the activity.

A technical effect of the aforementioned features is that it is advantageous in specificity, detailed methodology, quantifiable metrics, and practical applicability with minimum amount of data. This leads to more precise, reliable, and actionable outcomes in detecting the flow state. This provides accurate monitoring of attainment of the flow state for the activity performed by the user through a well-defined relationship and methodology, thus increasing the reliability and validity of detecting the flow state with the minimum amount of data. Beneficially, the aforementioned features can be used ranging from a simplest implementation to complex implementations, thus demonstrating that the flow index is identifiable even in its most basic form, using the minimum amount of data. Advantageously, incorporating additional data sources significantly enhances the accuracy and the reliability of the flow index.

A technical effect of determining the at least one of: the focus index, the stress index, the fatigue index is that precise and dynamic information about the user's movements, and/or orientation or position for an enhanced user experience, are provided.

Optionally, the fatigue index depends on the activity the user is engaged in. Optionally, the fatigue index lies in a range from 0 to 1, 0 to 10, 0 to 25, 0 to 100, and similar. For example, when the fatigue index may lie in the range from 0 to 25, the higher values may indicate high levels of fatigue and the lower values may indicate low levels of fatigue. Hence, fatigue index of 2/20 may indicate low fatigue, and alternatively the fatigue index of 15/20 may indicate high fatigue. Optionally, the fatigue index and the flow index are inversely related to each other, wherein the user gradually becomes fatigued during a course of the activity. Hence, the user's ability to sustain the flow index could decrease.

Optionally, the method further comprises:
determining at least one of: a blink rate, a blink speed, a blink pattern, by processing the first sensor data; and
determining the fatigue index of the user, based at least on the at least one of: the blink rate, the blink speed, the blink pattern;
wherein the determining of the flow index of the user is based also on the fatigue index.

Throughout the present disclosure, the term *"blink rate"* refers to the number of blinks that occur within a specific unit of time. The term *"blink speed"* refers to a speed of an individual blink of the user's eyes. Moreover, it is measured as to how quickly the user's eyes open and close during each blink. The term *"blink pattern"* refers to a sequence, a timing, and/or a characteristic of the blink which are unique to the user's eyes. When determining the blink rate, the image processing algorithm analyses the first sensor data to determine to closure and reopening of eyelids, by identifying changes in at least one of: a position, the shape of the pupil of each eye. When determining the blink speed, the image processing algorithm analyses the first sensor data to determine a beginning and ending of each blink of the user's eyes by analysing the corneal reflections of light emanating from the real-world environment from the surface of each eye. When determining the blink pattern, the image processing algorithm analyses the first sensor data to identify a start point and an end point of each blink, identify a duration of the blink, identify a regularity or irregularity of occurrences of the blink, and analyse a time interval between blinks.

Moreover, the fatigue index could be used to determine at least one of: tiredness, exhaustion, decreased performance by analysing at least one of: the blink rate, the blink speed, the blink pattern. Optionally, the fatigue index is directly related to the blink rate. Optionally, at least one blink pattern is pre-known to be indicative of fatigue, and an extent of similarity between the at least one blink pattern and the blink pattern that is determined, directly impacts the fatigue index. Optionally, the fatigue index is inversely related to the blink speed. In instances when the user is tired, at least one of: the blink rate is higher than usual, individual blinks are longer (i.e., it takes longer for the user to close and open the eyelids during an individual blink), there are flurries of rapid blinks every now and then when the user tries to awaken or refresh their eyes. For example, the blink rate may increase when the user is excited, aroused or angry. Moreover, temporal correlations could be analysed between at least one of: a blink rate, a blink speed, a blink pattern, and the at least one of: the pose of the user's head, the pose of the head-mounted device worn by the user on the user's head, the pose of the wearable device worn by the user, to infer cognitive load.

A technical effect of determining the fatigue index and using it for determining the flow index is that an experience of the user while performing the activity can be accommodated with varying levels of fatigue, which is used to determine capabilities and limitations of the user.

Optionally, the method, further comprises:
receiving calibration data from the eye-tracking means and the heart rate monitoring device, wherein the calibration data is collected during a calibration time period; and
determining a baseline focus index and a baseline stress index for the user, by processing the calibration data,
wherein the determining of the flow index of the user is based also on the baseline focus index and the baseline stress index.

Herein, the term *"calibration data"* refers to at least one of: measurements, parameters, signals obtained from the eye-tracking means and the heart rate monitoring device to establish a baseline specific to the user. Moreover, the term *"calibration time period"* refers to a duration in which the eye-tracking means and the heart rate monitoring device are calibrated. In other words, the calibration time period is a dedicated timeframe when the users are asked to perform the particular activity to facilitate collection of the calibration data, wherein the calibration time period may be a minute, 10 minutes, an hour, 5 hours, a day, and the like. A technical benefit of such calibration of the eye-tracking means and the heart rate monitoring device using the calibration data during the calibration time period, is that it ensures an accuracy and reliability in subsequent collection of the first sensor data and the second sensor data, wherein the eye-tracking means and the heart rate monitoring device can be adjusted based on the first sensor data and the second sensor data, respectively. A technical effect of incorporating the third sensor data, enhances a contextual relevance of the baseline focus index and the stress index that is determined during the calibration time period. This ensures that the flow index reflects a comprehensive understanding of the user's cognitive and physiological state, thus improving an overall accuracy and adaptability of the system.

Herein, the term *"baseline focus index"* refers to the user's typical or average level of focus observed during the calibration time period. Herein, the baseline focus index provides a reference for evaluating changes or variations in the levels of focus while being engaged in the activity. The term *"baseline stress index"* refers to the user's typical or initial level of stress observed during the calibration time period. Herein, the baseline stress index provides a reference for evaluating changes or variations in levels of stress while being engaged in the activity.

Subsequently, the flow index is determined by evaluating a relationship between the focus index and the stress index with respect to the baseline focus index and the baseline stress index, respectively. Herein, the focus index being greater than the baseline focus index indicates a focused state during the activity, which enables to attain the flow state. Conversely, the stress index being lesser than the baseline stress index indicates reduced stress, which contributes positively to the flow state.

Optionally, the method further comprises determining a baseline fatigue index for the user, by processing the calibration data,
wherein the determining of the flow index of the user is based also on the baseline fatigue index. Herein, the term *"baseline fatigue index"* refers to the user's typical or average level of fatigue observed during the calibration time period. Herein, the baseline fatigue index provides a reference for evaluating changes in the physiological responses (for example, such as the heart rate, heart rate variability) associated with fatigue. Subsequently, the flow index is further determined by evaluating a relationship between the fatigue index with respect to the baseline fatigue index. Herein, the fatigue index being greater than the baseline fatigue index indicates that the user has low fatigue during the activity, which contributes positively to the flow state.

Optionally, the method further comprises:
collecting user flow state data comprising flow indices of the user, that are determined over a first time period; and
determining a focus weight multiplier and a stress weight multiplier for the focus index and the stress index, respectively, by analyzing the user flow state data for identifying how the focus index and the stress index impact the determination of the flow indices,
wherein the determining of the flow index of the user is based also on the focus weight multiplier and the stress weight multiplier.

Throughout the present disclosure the term *"user flow state data"* refers to the information that capture the user's performance while being engaged in the particular activity. The user flow state data is captured using the eye-tracking means and the heart rate monitoring device during the first time period, wherein the user flow state data comprises measurement related to focus and stress of the user while being engaged in the activity. Herein, the term *"first time period"* refers to a duration in which the user is engaged in the activity, wherein the first time period may be an hour, 2 hours, 5 hours, 10 hours, 20 hours, a day, a week, and the like. Throughout the first time period, individual flow indices are determined periodically or at particular time intervals within the first time period, which are collected (namely, aggregated) to form the user flow state data representing the user's flow state experience over the first time period. A technical effect of collecting the user flow state data is to gather insights into the user's patterns in experiencing the flow state during the first time period, and enables analysing fluctuations in the mental state of the user. A technical effect of determining the focus weight multiplier and the stress weight multiplier based on the user flow state data, combined with the third sensor data, enables a dynamic adjustment of the flow index. This enhances an ability of the system to accurately reflect the user's cognitive and emotional states by accounting for both historical patterns and real-time contextual factors.

Moreover, the term *"focus weight multiplier"* refers to a relative weight assigned to the focus index in influencing a calculation of the flow indices. The term *"stress weight multiplier"* refers to another relative weight assigned to the stress index in influencing a calculation of the stress indices. The focus weight multiplier and the stress weight multiplier could be coefficients or factors which are used to adjust a contribution of focus index and stress index, respectively, in determining the overall flow index for the user. Optionally, the focus weight multiplier and the stress weight multiplier are determined using any one of: a statistical analysis, an algorithm, a modelling technique. Thereafter the focus weight multiplier and the stress weight multiplier are used in subsequent calculations to adjust an impact of the focus index and the stress index on determination of the flow indices. For example, when the focus weight multiplier is high, it indicates that variations in the focus index has a strong influence on the flow indices. When the stress weight multiplier is high, it indicates that variations in the stress index has a strong influence on the flow indices. An exemplary formula (2) for determining the flow index based also on the focus weight multiplier and the stress weight multiplier is given by, $Flow Index = a ∗ \frac{Focus Index}{Baseline Focus Index} - b ∗ \frac{Stress Index}{Baseline Stress Index}$ wherein 'a' denotes the focus weight multiplier and 'b' denotes the stress weight multiplier. A technical effect of determining the focus weight multiplier and the stress weight multiplier, it facilitates personalizing an assessment of the flow state for each user, which enhances an accuracy of the flow index.

Optionally, the method further comprises determining a fatigue weight multiplier for the fatigue index, respectively, by analysing the user flow state data for identifying how the fatigue index impact the determination of the flow indices,
wherein the determining of the flow index of the user is based also on the fatigue weight multiplier.

Herein, the term *"fatigue weight multiplier"* refers to a relative weight assigned to the focus index in influencing a calculation of the flow indices. The fatigue weight multiplier could also be coefficients or factors which are used to adjust a contribution of the fatigue index, in determining the overall flow index for the user. The fatigue weight multiplier is determined in a manner similar to a manner of determining the focus weight multiplier and the stress weight multiplier. Thereafter the fatigue weight multiplier are used in subsequent calculations to adjust an impact of the fatigue index on determination of the flow indices. When the fatigue weight multiplier is high, it indicates that elevated levels of fatigue has a pronounced effect on influencing the user's ability to attain the flow state. Continuing with the exemplary formula (2), a formula (3) for determining the flow index based also on the fatigue weight multiplier is given by, $Flow Index = a ∗ \frac{Focus Index}{Baseline Focus Index} - b ∗ \frac{Stress Index}{Baseline Stress Index} - c ∗ \frac{Fatigue Index}{Baseline Fatigur Index}$ wherein, 'c' denotes the fatigue index multiplier.

Optionally, the method further comprises at least one of:
generating a visualization indicative of at least the focus index, the stress index, and the flow index, and providing the visualization on an interactive user interface;
determining the flow index for a second time period, and providing a representation of the flow index for the second time period on an interactive user interface; and
determining a third time period in a given day for which the flow index is higher than a predefined value, comparing the third time period with a target flow state time period for generating a comparison result, and providing, on an interactive user interface, a representation of at least one of: the third time period, the comparison result.

In this regard, the visualization is generated using at least one data visualization technique, using the determined values of the focus index, the stress index, and the flow index. Such data visualization techniques are well known in the art. Examples of the visualization include, but are not limited to, a tilted four axis, a bar graph, a line diagram, a pie chart, a radial graph, a table, and a list. Optionally, the visualization generated is also indicative of the fatigue index. A technical effect of generating the visualization and its presentation on the interactive user interface is that they enable the user to easily comprehend the values of the focus index, the stress index, and the flow index, and to conveniently track changes in such values over a period of time. A technical effect of incorporating the third sensor data into the generation of the visualization indicative of at least the focus index, the stress index, and the flow index, enhances a contextual relevance and accuracy of the presented visualization on the interactive user interface. This allows users to better understand how their physical interactions and posture influence their focus, stress, and flow states, thereby enabling more informed decisions for optimizing their performance.

Herein, the interactive user interface is displayed on a display device. The display device is associated with (or used by) the user, and is capable of enabling the user to perform specific tasks associated with the aforementioned method. Furthermore, the display device is intended to be broadly interpreted to include any electronic device that may be used to facilitate interaction of the user using it with the system. this interaction may be facilitated by one or more of a touch-sensitive screen of the display device, buttons on the display device, microphone on the display device, or similar. The term *"interactive user interface"* relates to a structured set of interactive user interface elements rendered on a display of the display device. Optionally, the interactive user interface is generated by any collection or set of instructions executable by the at least one processor. Additionally, the interactive user interface is operable to interact with the user to convey graphical and/or textual information and receive input from the user. Furthermore, the interactive user interface elements refer to visual objects that have a size and position in the interactive user interface, and serve as a means of interacting with the person with respect to order picking. A given user interface element could be used to present/display an output, receive an input, or perform a combination of these. Text blocks, labels, text boxes, list boxes, lines, images windows, dialog boxes, frames, panels, menus, buttons, icons, statistical representations, and the like, are examples of interactive user interface elements. In addition to size and position, the interactive user interface element may have other properties, such as a margin, spacing, or the like.

Subsequently, the flow index is determined for the second time period, wherein the second time period is different from the first time period. This is used to determine changes in the flow state of the ser, which evolves across different activities. In this regard, if any adjustments have been made to the user's environment, activity or interactions based on the flow index determined during the first time period, then determining the flow index for the second time period facilitates in evaluating effectiveness of these adjustments.

Thereafter, the third time period is determined to be a particular timeframe in the given day, in which the flow index is higher than the predefined value, wherein the third time period represents a time when the user experiences a relatively high level of engagement, focus or experience. Optionally, the term *"predefined value"* refers to an ideal timeframe during which the user aims to attain the flow state. In an instance, when the flow state of the user is attained, the predefined value lies in a range of 75 to 100, on a scale of 1-100. The predefined value, for example, may lie in a range of 75, 80, or 90 to 85, 95, or 100, on the scale of 1-100. In another instance, the flow state could be attained at a particular time of the day. The predefined value in such an instance is unique for the user, as said user may desire to attain the flow state at different times throughout the day. For example, the user may desire to attain a given flow state during morning, between 9 a.m. to 11 a.m., and another given flow state during afternoon, between 2 p.m. to 4 p.m.

Subsequently, the comparison result is generated. Herein, the term *"comparison result"* refers to at least one of: a metric, a statistic, a qualitative evaluation which indicates how closely the third time period aligns with the target flow state time period. The at least one of: the third time period, the comparison result are represented visually or descriptively on the interactive user interface. This representation could be in a form of, but not limited to, another bar graph, another line diagram, another pie chart, another radial graph, another table, and another a list.

A technical effect of determining a third time period in the given day is to assess how well the third time period matches or deviates from the target flow state time period, which facilitates in understanding when the user as attained the flow state while engaged in the activity.

Optionally, the method further comprises:
providing, via an interactive user interface, a timer;
receiving a user input indicative of a time duration that is set by the user on the timer, wherein the user is engaged in the activity for the time duration;
generating a control signal for the timer to ring upon elapsing of the time duration; and
upon elapsing of the time duration, generating a flow state report indicative of at least one of: the flow index, a status of the user attaining the flow state for the activity, the focus index, the stress index, during the time duration.

In this regard, the timer is provided via the interactive user interface to enable the user to set the time duration for the activity. The interactive user interface displays a digital representation of the timer, wherein the timer displays time in seconds, minutes, hours. This allows the user to monitor a passage of time. Optionally, the timer is implemented as a pomodoro timer. In this regard, the user sets the time duration by interacting with the timer on the interactive user interface, wherein such interaction can be performed by typing the time duration, selecting the time duration using interactive controls, and similar. The time duration is set by the user dynamically, based on the activity they ae engaged in. Upon setting the time duration on the timer, the user engages in the activity for said time duration. Herein, the timer runs and optionally displays a countdown, indicating the elapsing of the time duration, wherein the time duration elapses when the timer either reaches zero or the time duration that was set.

Subsequently, when the time duration elapses, the control signal is generated to trigger the timer to ring, which provides a prompt to the user indicating a completion of their allocated time duration for the activity. Herein, the control signal activates an alert, a visual notification, an audible sound to indicate that the time duration has concluded. When the timer reaches the time duration, the flow state report is generated which is optionally displayed on the interactive user interface. Herein, the flow state report summarizes engagement metrics during the time duration, wherein the flow state report provides insights into at least one of: experience, the level of focus, the level of stress, whether the flow state was achieved while engaging in the activity for the time duration. The flow state report is provided to the user, via the interactive user interface, thereby enabling the user to easily view and comprehend their flow state during the activity, which further enables the user to further improve their flow state when performing the activity, or other activities, in future. A technical effect of generating the flow state report in such a manner is that the user receives a detailed assessment of their engagement and mental state during the time duration, which is valuable for at least one of: self-assessment, time management, adjust their performance in the activity. A technical effect of the flow state report comprising the third sensor data enhances an accuracy and a depth of assessment by incorporating physical context into the determination of at least one of: the flow index, the focus index, the stress index. This enables the user to gain more precise insights into how their physical state and posture influenced their focus, stress, and flow during the activity, supporting better self-assessment and performance optimization.

Optionally, the method further comprises:
determining whether the stress index exceeds its predefined acceptable threshold;
when it is determined that the stress index exceeds its predefined acceptable threshold, generating a recommendation to perform a task, wherein the task, when performed by the user, enables lowering of the stress index; and
providing a representation of the recommendation on an interactive user interface.

In this regard, the stress index of the user is continuously monitored to determine how stressed the user is while being engaged in the activity. Herein, the term *"predefined acceptable threshold"* refers to a maximum level of stress that is acceptable or can be tolerated when the user is engaged in the activity. Optionally, the predefined acceptable threshold lies in a range of 80 to 100, on a scale of 0-100. When the stress index exceeds its predefined acceptable threshold, it indicates that the level of stress of the user has surpassed acceptable limits. It will be appreciated that such levels of stress (i.e., when the level of stress of the user surpasses the acceptable limits), is beneficial in a short duration of time, but becomes harmful when the stress prolongs for a long duration of time. When the stress index exceeds its predefined acceptable threshold, the recommendations may be generated in a form of notifications, alerts, interventions, adaptive responses and the like to mitigate the level of stress of the user. Such recommendations are used to perform the task to lower the stress index, wherein the recommendations are effective in promoting relaxation, reducing anxiety, calming the user's state of mind. The tasks could vary depending on preferences of the user, options available, and a context in which the level of stress is to be managed. Examples of the task may include, but are not limited to, deep breathing exercises, guided meditation, physical exercises, mindfulness practices, engaging in hobbies, and similar. Subsequently, upon performing the task, the user might experience a reduction in the levels of stress, which consequently lowers the stress index. The recommendation is represented via the interactive user interface, to provide the user a quick understanding of what the task involves and how to implement said task. The representation of the recommendation may be in a form of at least one of: visual instructions (for example, such as images, graphics, animations), audio instructions, textual instructions, interactive controls (for example, such as buttons, sliders, progress bars), progress tracking, background information, and similar.

In an exemplary implementation, the stress index may be measured to be 85 (i.e., high stress) for the long duration of time, i.e., at least 60 minutes. Hence, the stress index exceeds its predefined acceptable threshold. A visualization may be generated, wherein the visualization is indicative of this stress index, and wherein the visualization may be a graph. Herein, the stress index for the duration may be highlighted in red colour to draw attention to them on the graph. In instances where the stress index may be measured to be 85 for the short duration of time, i.e., for 5 minutes, such stress index may not be highlighted in any colour. Herein, if the user receives notifications regarding the high stress at real time or near-real time, it may cause more stress to the user. Hence, the user may receive a daily summary of the stress index, wherein periods of high stress for the long duration of time may be highlighted, and a recommendation (for example, such as advice on a length of a break that should be taken) may be suggested to perform a task to avoid it in the future.

As an example, the recommendation may be the guided meditation. Hence, the representation of the recommendation that is provided on the interactive user interface involves illustrations of meditation postures, illustrations of breathing exercises, an audio guiding the user through the breathing exercises, textual instructions on breathing patterns.

A technical benefit of regulating the stress index in such a manner is that it enables improvement of an overall well-being of the user, while facilitating stress management in real time or in near-real time. A technical effect of incorporating the third sensor data into the determination of the stress index enhances a precision of detecting stress levels and tailoring recommendations. This allows the system to provide context-aware tasks that more effectively reduce stress, thereby improving the user's overall well-being and promoting real-time stress management with increased accuracy.

Optionally, the method further comprises creating, if the user has attained the flow state for the activity, a second control signal to control at least one communication means associated with the user. The second control signal can be used to, for example to enable or disable different functions of the at least one communication means. In an example, the at least one communication means could be a phone, a tablet, a smartwatch, and the like. As an example, the second control signal can be used to turn audio alerts off from the phone of the user when the user is in the flow state. Additionally, when not in the flow state, method comprises creating a third control signal to turn alerts on from a phone of the user (or in general turn on functions which have been disabled with the second control signal).

A technical effect of controlling the at least one communication means associated with the user (which has attained the flow state of the activity) is that the user does not have to control the at least one communication means manually because the control is automatic. Furthermore, manual control of the communication means would change the flow state of the user This way external devices or system do not disturb the user when in flow state. A technical effect of integrating the third sensor data in determining the flow state enhances a reliability of identifying when the user has attained the flow state. This ensures that the automated control of communication means is contextually accurate, minimizing disruptions and maintaining the user's focus, thereby preserving the flow state effectively without requiring manual intervention.

The present disclosure also relates to the second aspect as described above. Various embodiments and variants disclosed above, with respect to the aforementioned first aspect, apply *mutatis mutandis* to the second aspect.

Optionally, the at least one processor is further configured to:
determine at least one of: a blink rate, a blink speed, a blink pattern, by processing the first sensor data; and
determine the fatigue index of the user, based at least on the at least one of: the blink rate, the blink speed, the blink pattern;
wherein the determining of the flow index of the user is based also on the fatigue index.

A technical effect of determining the fatigue index and using it for determining the flow index is that an experience of the user while performing the activity can be accommodated with varying levels of fatigue, which is used to determine capabilities and limitations of the user.

Optionally, the at least one processor is further configured to:
receive calibration data from the eye-tracking means and the heart rate monitoring device, wherein the calibration data is collected during a calibration time period; and
determine a baseline focus index and a baseline stress index for the user, by processing the calibration data,
wherein the determining of the flow index of the user is based also on the baseline focus index and the baseline stress index.

A technical benefit of such calibration of the eye-tracking means and the heart rate monitoring device using the calibration data during the calibration time period, is that it ensures an accuracy and reliability in subsequent collection of the first sensor data and the second sensor data, wherein the eye-tracking means and the heart rate monitoring device can be adjusted based on the first sensor data and the second sensor data, respectively. A technical effect of incorporating the third sensor data, enhances a contextual relevance of the baseline focus index and the stress index that is determined during the calibration time period. This ensures that the flow index reflects a comprehensive understanding of the user's cognitive and physiological state, thus improving an overall accuracy and adaptability of the system.

Optionally, the at least one processor is further configured to perform at least one of:
generate a visualization indicative of at least the focus index, the stress index, and the flow index, and providing the visualization on an interactive user interface;
determine the flow index for a second time period, and providing a representation of the flow index for the second time period on an interactive user interface; and
determine a third time period in a given day for which the flow index is higher than a predefined value, comparing the third time period with a target flow state time period for generating a comparison result, and providing, on an interactive user interface, a representation of at least one of: the third time period, the comparison result.

A technical effect of generating the visualization and its presentation on the interactive user interface is that they enable the user to easily comprehend the values of the focus index, the stress index, and the flow index, and to conveniently track changes in such values over a period of time. A technical effect of determining a third time period in the given day is to assess how well the third time period matches or deviates from the target flow state time period, which facilitates in understanding when the user as attained the flow state while engaged in the activity. A technical effect of incorporating the third sensor data into the generation of the visualization indicative of at least the focus index, the stress index, and the flow index, enhances a contextual relevance and accuracy of the presented visualization on the interactive user interface. This allows users to better understand how their physical interactions and posture influence their focus, stress, and flow states, thereby enabling more informed decisions for optimizing their performance.

Optionally, the at least one processor is further configured to:
provide, via an interactive user interface, a timer;
receive a user input indicative of a time duration that is set by the user on the timer, wherein the user is engaged in the activity for the time duration;
generate a control signal for the timer to ring upon elapsing of the time duration; and
upon elapsing of the time duration, generate a flow state report indicative of at least one of: the flow index, a status of the user attaining the flow state for the activity, the focus index, the stress index, during the time duration.

A technical effect of generating the flow state report in such a manner is that the user receives a detailed assessment of their engagement and mental state during the time duration, which is valuable for at least one of: self-assessment, time management, adjust their performance in the activity. A technical effect of the flow state report comprising the third sensor data enhances an accuracy and a depth of assessment by incorporating physical context into the determination of at least one of: the flow index, the focus index, the stress index. This enables the user to gain more precise insights into how their physical state and posture influenced their focus, stress, and flow during the activity, supporting better self-assessment and performance optimization.

Optionally, the at least one processor is further configured to:
determine whether the stress index exceeds its predefined acceptable threshold;
when it is determined that the stress index exceeds its predefined acceptable threshold, generate a recommendation to perform a task, wherein the task, when performed by the user, enables lowering of the stress index; and
provide a representation of the recommendation on an interactive user interface.

A technical benefit of regulating the stress index in such a manner is that it enables improvement of an overall well-being of the user, while facilitating stress management in real time or in near-real time. A technical effect of incorporating the third sensor data into the determination of the stress index enhances a precision of detecting stress levels and tailoring recommendations. This allows the system to provide context-aware tasks that more effectively reduce stress, thereby improving the user's overall well-being and promoting real-time stress management with increased accuracy.

### DETAILED DESCRIPTION OF THE DRAWINGS

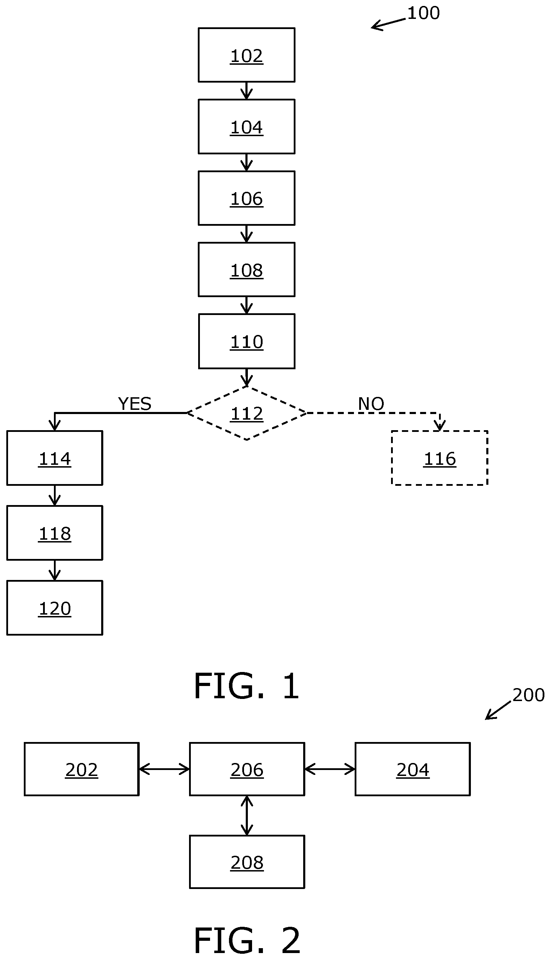

Referring to FIG. 1, illustrated are steps of a method for monitoring attainment of a flow state for an activity by a user, in accordance with an embodiment of the present disclosure. At step **102,** a first sensor data is received from an eye-tracking means, and a second sensor data is received from a heart rate monitoring device, wherein the first sensor data and the second sensor data are collected while the user is engaged in the activity. At step **104,** an eye fixation frequency and an eye fixation percentage are determined by processing the first sensor data, and a heart rate and a heart rate variability are determined by processing the second sensor data. At step **106,** a focus index of the user is determined, based on the eye fixation frequency and the eye fixation percentage. At step **108,** a stress index of the user is determined, based on the heart rate and the heart rate variability. At step **110,** a flow index of the user is determined, based on a difference between the focus index and the stress index. Optionally, at step **112,** it is determined whether the determined flow index is equal to or greater than a predefined flow index for the activity. At step **114,** it is detected that the user has attained the flow state for the activity, when the determined flow index is equal to or greater than a predefined flow index for the activity at the step **112.** Alternatively, optionally, at step **116,** it is detected that the user has not attained the flow state for the activity, when the determined flow index is lesser than the predefined flow index for the activity at the step **112.** At step **118,** a third sensor data is received from a tracking means. At step **120,** at least one of: a pose of the user's head, a pose of a head-mounted device worn by the user on the user's head, a pose of a user-interaction controller that is held or worn by the user, is determined, by processing the third sensor data, wherein the at least one of: the pose of the user's head, the pose of the head-mounted device worn by the user on the user's head, the pose of the wearable device worn by the user, is utilized when determining at least one of: the focus index, the stress index , a fatigue index.

The aforementioned steps are only illustrative and other alternatives can also be provided where one or more steps are added, one or more steps are removed, or one or more steps are provided in a different sequence without departing from the scope of the claims herein.

Referring to FIG. 2, illustrated is an architecture of a system **200** for monitoring attainment of a flow state for an activity by a user, in accordance with an embodiment of the present disclosure. The system **200** comprises an eye-tracking means **202,** a heart rate monitoring device **204,** at least one processor (depicted as a processor **206),** and a tracking means **208.** The processor **206** is communicably coupled to the eye-tracking means **202,** the heart rate monitoring device **204,** and the tracking means **208.** The processor **206** is configured to implement processing steps of a method for monitoring attainment of the flow state for the activity by the user.

It may be understood by a person skilled in the art that the FIG. 2 includes a simplified architecture of a systems **200** for sake of clarity, which should not unduly limit the scope of the claims herein. The person skilled in the art will recognize many variations, alternatives, and modifications of embodiments of the present disclosure.

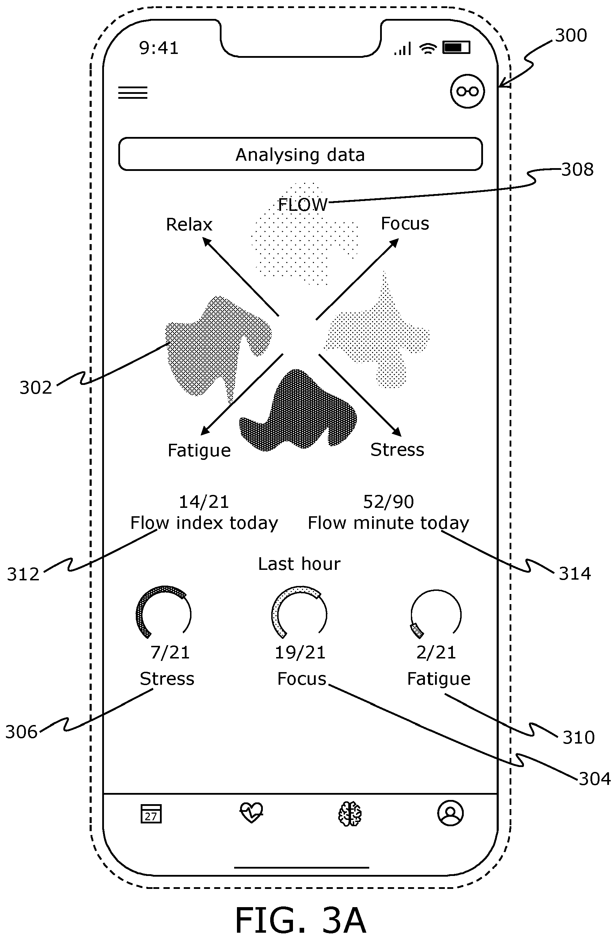

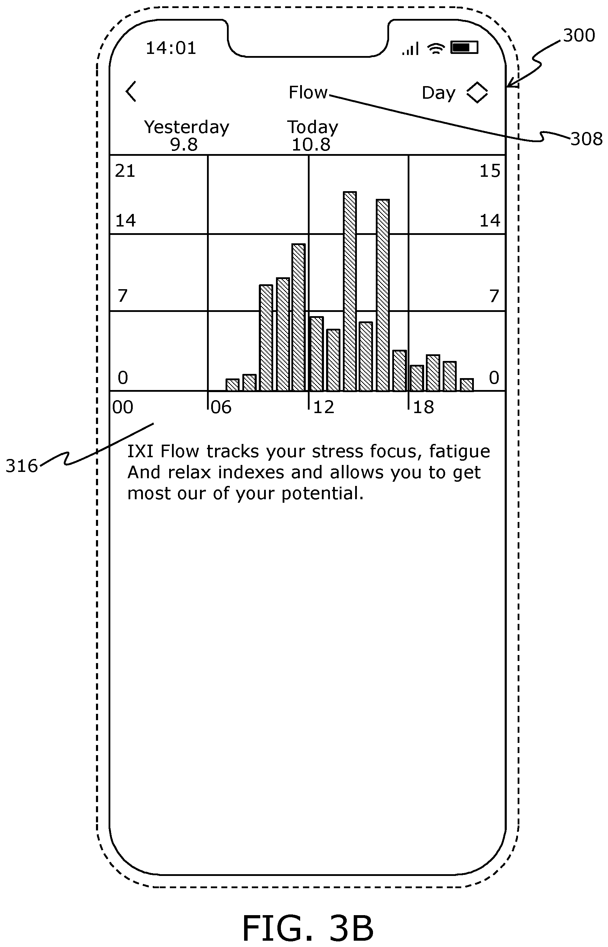

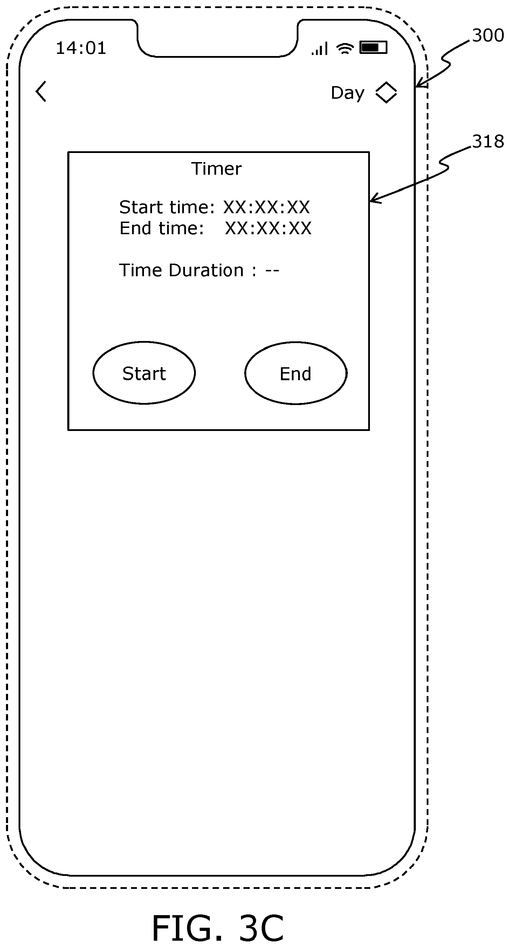

Referring to FIGs. 3A, 3B, and 3C, illustrated are various views of an interactive user interface **300,** in accordance with an embodiment of the present disclosure. In FIG. 3A, a visualization **302** is provided on the interactive user interface **300,** the visualization **302** being indicative of at least a focus index **304,** a stress index **306,** and a flow index **308.** Optionally, the visualization **302** is also indicative of the fatigue index **310.** In this regard, the visualization **302** is in a form of a tilted four axis. The flow index **308** is determined for a second time period, wherein a representation **312** of the flow index **308** for the second time period is provided on the interactive user interface **300.** Thereafter, a third time period in a given day for which the flow index is higher than a predefined value is determined, wherein the third time period with a target flow state time period is used for generating a comparison result, to provide, on the interactive user interface **300,** a representation **314** of at least one of: the third time period, the comparison result. Alternatively, in FIG. 3B, a visualization **316** is provided on the interactive user interface **300,** the visualization **316** being indicative of the flow index **308** over a period of time. The visualization **316** is in a form of a bar graph, wherein the horizontal axis represents time of a given day, and the vertical axis represents a range of the flow index. In FIG. 3C, a timer **318** is provided on the interactive user interface **300.** A user input indicative of a time duration is set by a user on the timer **314,** wherein the user is engaged in an activity for the time duration.

## Claims

1. A computer implemented method for monitoring attainment of a flow state for an activity by a user, the method comprising:
receiving first sensor data from an eye-tracking means (202), and second sensor data from a heart rate monitoring device (204), wherein the first sensor data and the second sensor data are collected while the user is engaged in the activity;
determining an eye fixation frequency and an eye fixation percentage by processing the first sensor data, and determining a heart rate and a heart rate variability by processing the second sensor data;
determining a focus index (304) of the user, based on the eye fixation frequency and the eye fixation percentage;
determining a stress index (306) of the user, based on the heart rate and the heart rate variability;
determining a flow index (308) of the user, based on a difference between the focus index and the stress index;
detecting that the user has attained the flow state for the activity, when the determined flow index is equal to or greater than a predefined flow index for the activity;
receiving third sensor data from a tracking means (208); and
determining at least one of: a pose of the user's head, a pose of a head-mounted device worn by the user on the user's head, a pose of a user-interaction controller that is held or worn by the user, by processing the third sensor data,
wherein the at least one of: the pose of the user's head, the pose of the head-mounted device worn by the user on the user's head, the pose of the user-interaction controller worn by the user, is utilized when determining at least one of: the focus index (304), the stress index (306), a fatigue index (310).

2. The method of claim 1, further comprising:
determining at least one of: a blink rate, a blink speed, a blink pattern, by processing the first sensor data; and
determining the fatigue index (310) of the user, based at least on the at least one of: the blink rate, the blink speed, the blink pattern; wherein the determining of the flow index (308) of the user is based also on the fatigue index.

3. The method of any of the preceding claims, further comprising:
receiving calibration data from the eye-tracking means (202) and the heart rate monitoring device (204), wherein the calibration data is collected during a calibration time period; and
determining a baseline focus index and a baseline stress index for the user, by processing the calibration data,
wherein the determining of the flow index (308) of the user is based also on the baseline focus index and the baseline stress index.

4. The method of any of the preceding claims, further comprising:
collecting user flow state data comprising flow indices of the user, that are determined over a first time period; and
determining a focus weight multiplier and a stress weight multiplier for the focus index (304) and the stress index (306), respectively, by analyzing the user flow state data for identifying how the focus index and the stress index impact the determination of the flow indices,
wherein the determining of the flow index (308) of the user is based also on the focus weight multiplier and the stress weight multiplier.

5. The method of any of the preceding claims, further comprising at least one of:
generating a visualization (302, 316) indicative of at least the focus index (304), the stress index (306), and the flow index (308), and providing the visualization on an interactive user interface (300);
determining the flow index for a second time period, and providing a representation (312) of the flow index for the second time period on an interactive user interface; and
determining a third time period in a given day for which the flow index is higher than a predefined value, comparing the third time period with a target flow state time period for generating a comparison result, and providing, on an interactive user interface, a representation (314) of at least one of: the third time period, the comparison result.

6. The method of any of the preceding claims, further comprising:
providing, via an interactive user interface (300), a timer (318);
receiving a user input indicative of a time duration that is set by the user on the timer, wherein the user is engaged in the activity for the time duration;
generating a control signal for the timer to ring upon elapsing of the time duration; and
upon elapsing of the time duration, generating a flow state report indicative of at least one of: the flow index (308), a status of the user attaining the flow state for the activity, the focus index (304), the stress index (306), during the time duration.

7. The method of any of the preceding claims, further comprising:
determining whether the stress index (306) exceeds its predefined acceptable threshold;
when it is determined that the stress index exceeds its predefined acceptable threshold, generating a recommendation to perform a task, wherein the task, when performed by the user, enables lowering of the stress index; and
providing a representation of the recommendation on an interactive user interface (300).

8. A method according to any of the preceding claims, further comprising, if the user has attained the flow state for the activity, creating a second control signal to control at least one communication means associated with the user.

9. A system (200) for monitoring attainment of a flow state for an activity by a user, the system comprising:
an eye-tracking means (202);
a heart rate monitoring device (204);
a tracking means (208); and
at least one processor (206) configured to:
receive first sensor data from the eye-tracking means, and second sensor data from the heart rate monitoring device, wherein the first sensor data and the second sensor data are collected while the user is engaged in the activity;
determine an eye fixation frequency and an eye fixation percentage by processing the first sensor data, and determining a heart rate and a heart rate variability by processing the second sensor data;
determine a focus index (304) of the user, based on the eye fixation frequency and the eye fixation percentage;
determine a stress index (306) of the user, based on the heart rate and the heart rate variability;
determine a flow index (308) of the user, based on a difference between the focus index and the stress index;
detect that the user has attained the flow state for the activity, when the determined flow index is equal to or greater than a predefined flow index for the activity;
receive third sensor data from the tracking means; and
determine at least one of: a pose of the user's head, a pose of a head-mounted device worn by the user on the user's head, a pose of a user-interaction controller that is held or worn by the user, by processing the third sensor data,
wherein the at least one of: the pose of the user's head, the pose of the head-mounted device worn by the user on the user's head, the pose of the user-interaction controller worn by the user, is utilized when determining at least one of: the focus index (304), the stress index (306), a fatigue index (310).

10. The system of claim 9, wherein the at least one processor (206) is further configured to:
determine at least one of: a blink rate, a blink speed, a blink pattern, by processing the first sensor data; and
determine a fatigue index (310) of the user, based at least on the at least one of: the blink rate, the blink speed, the blink pattern; wherein the determining of the flow index (308) of the user is based also on the fatigue index.

11. The system of any of claims 9 or 10, wherein the at least one processor (206) is further configured to:
receive calibration data from the eye-tracking means (202) and the heart rate monitoring device (204), wherein the calibration data is collected during a calibration time period; and
determine a baseline focus index and a baseline stress index for the user, by processing the calibration data,
wherein the determining of the flow index (308) of the user is based also on the baseline focus index and the baseline stress index.

12. The system of any of claims 9-11, wherein the at least one processor (206) is further configured to perform at least one of:
generate a visualization (302, 316) indicative of at least the focus index (304), the stress index (306), and the flow index (308), and providing the visualization on an interactive user interface (300);
determine the flow index for a second time period, and providing a representation (312) of the flow index for the second time period on an interactive user interface; and
determine a third time period in a given day for which the flow index is higher than a predefined value, comparing the third time period with a target flow state time period for generating a comparison result, and providing, on an interactive user interface, a representation (314) of at least one of: the third time period, the comparison result.

13. The system of any of claims 9-12, wherein the at least one processor (206) is further configured to:
provide, via an interactive user interface (300), a timer (318);
receive a user input indicative of a time duration that is set by the user on the timer, wherein the user is engaged in the activity for the time duration;
generate a control signal for the timer to ring upon elapsing of the time duration; and
upon elapsing of the time duration, generate a flow state report indicative of at least one of: the flow index (308), a status of the user attaining the flow state for the activity, the focus index (304), the stress index (306), during the time duration.

14. The system of any of claims 9-13, wherein the at least one processor (206) is further configured to:
determine whether the stress index (306) exceeds its predefined acceptable threshold;
when it is determined that the stress index exceeds its predefined acceptable threshold, generate a recommendation to perform a task, wherein the task, when performed by the user, enables lowering of the stress index; and
provide a representation of the recommendation on an interactive user interface (300).

## Patentansprüche

1. Computerimplementiertes Verfahren zur Überwachung des Erreichens eines Flow-Zustands für eine Aktivität durch einen Benutzer, wobei das Verfahren umfasst:
Empfangen von ersten Sensordaten von einem Blickverfolgungsmittel (202) und zweiten Sensordaten von einer Herzfrequenzüberwachungsvorrichtung (204), wobei die ersten Sensordaten und die zweiten Sensordaten erhoben werden, während der Benutzer die Aktivität ausführt;
Bestimmen einer Blickfixierungsfrequenz und eines Blickfixierungsprozentsatzes durch Verarbeiten der ersten Sensordaten, und Bestimmen einer Herzfrequenz und einer Herzfrequenzvariabilität durch Verarbeiten der zweiten Sensordaten;
Bestimmen eines Fokusindex (304) des Benutzers basierend auf der Blickfixierungsfrequenz und dem Blickfixierungsprozentsatz;
Bestimmen eines Stressindex (306) des Benutzers, basierend auf der Herzfrequenz und der Herzfrequenzvariabilität;
Bestimmen eines Flow-Index (308) des Benutzers, basierend auf einer Differenz zwischen dem Fokusindex und dem Stressindex;
Erfassen, dass der Benutzer den Flow-Zustand für die Aktivität erreicht hat, wenn der bestimmte Flow-Index gleich oder größer als ein vordefinierter Flow-Index für die Aktivität ist;
Empfangen von dritten Sensordaten von einem Verfolgungsmittel (208); und
Bestimmen von wenigstens einem von: einer Kopfhaltung des Benutzers, einer Haltung einer am Kopf montierten Vorrichtung, die von dem Benutzer am Kopf des Benutzers getragen wird, einer Haltung einer Benutzerinteraktionssteuerung, die von dem Benutzer gehalten oder getragen wird, durch Verarbeiten der dritten Sensordaten,
wobei das mindestens eine von: der Kopfhaltung des Benutzers, der Haltung der kopfmontierten Vorrichtung, die von dem Benutzer am Kopf des Benutzers getragen wird, der Haltung der von dem Benutzer getragenen Benutzerinteraktionssteuerung verwendet wird, wenn mindestens eines bestimmt wird von: dem Fokusindex (304), dem Stressindex (306), einem Ermüdungsindex (310).

2. Verfahren nach Anspruch 1, ferner umfassend:
Bestimmen von mindestens einem von: einer Lidschlagrate, einer Lidschlaggeschwindigkeit, einem Lidschlagmuster, durch Verarbeiten der ersten Sensordaten; und
Bestimmen des Ermüdungsindex (310) des Benutzers, basierend mindestens auf dem mindestens einen von: der Lidschlagrate, der Lidschlaggeschwindigkeit, dem Lidschlagmuster; wobei das Bestimmen des Flow-Index (308) des Benutzers auch auf dem Ermüdungsindex basiert.

3. Verfahren nach einem der vorstehenden Ansprüche, ferner umfassend:
Empfangen von Kalibrierungsdaten von dem Blickverfolgungsmittel (202) und der Herzfrequenzüberwachungsvorrichtung (204), wobei die Kalibrierungsdaten während einer Kalibrierungszeitspanne erhoben werden; und
Bestimmen eines Basislinienfokusindex und eines Basislinienstressindex für den Benutzer durch Verarbeiten der Kalibrierungsdaten, wobei das Bestimmen des Flow-Index (308) des Benutzers auch auf dem Basislinienfokusindex und dem Basislinienstressindex basiert.

4. Verfahren nach einem der vorstehenden Ansprüche, ferner umfassend:
Erheben von Benutzer-Flow-Zustandsdaten, die Flow-Indizes des Benutzers umfassen, die über eine erste Zeitspanne bestimmt werden; und
Bestimmen eines Fokusgewichtsmultiplikators und eines Stressgewichtsmultiplikators für den Fokusindex (304) bzw. den Stressindex (306) durch Analysieren der Benutzer-Flow-Zustandsdaten zum Identifizieren, wie der Fokusindex und der Stressindex die Bestimmung der Flow-Indizes beeinflussen, wobei das Bestimmen des Flow-Index (308) des Benutzers auch auf dem Fokusgewichtsmultiplikator und dem Stressgewichtsmultiplikator basiert.

5. Verfahren nach einem der vorstehenden Ansprüche, ferner umfassend mindestens eines von:
Erzeugen einer Visualisierung (302, 316), die mindestens den Fokusindex (304), den Stressindex (306) und den Flow-Index (308) angibt, und Bereitstellen der Visualisierung auf einer interaktiven Benutzerschnittstelle (300);
Bestimmen des Flow-Index für eine zweite Zeitspanne und Bereitstellen einer Darstellung (312) des Flow-Index für die zweite Zeitspanne auf einer interaktiven Benutzerschnittstelle; und
Bestimmen einer dritten Zeitspanne an einem bestimmten Tag, für die der Flow-Index höher als ein vordefinierter Wert ist, Vergleichen der dritten Zeitspanne mit einer Ziel-Flow-Zustandszeitspanne zum Erzeugen eines Vergleichsergebnisses, und Bereitstellen, auf einer interaktiven Benutzerschnittstelle, einer Darstellung (314) von mindestens einem von: der dritten Zeitspanne, dem Vergleichsergebnis.

6. Verfahren nach einem der vorstehenden Ansprüche, ferner umfassend:
Bereitstellen, über eine interaktive Benutzerschnittstelle (300), eines Zeitgebers (318);
Empfangen einer Benutzereingabe, die eine Zeitdauer angibt, die von dem Benutzer an dem Zeitgeber eingestellt wird, wobei der Benutzer die Aktivität für die Zeitdauer ausführt;
Erzeugen eines Steuersignals für den Zeitgeber, um nach Ablauf der Zeitdauer zu klingeln; und
nach Ablauf der Zeitdauer, Erzeugen eines Flow-Zustandsberichts, der mindestens eines angibt von: dem Flow-Index (308), einem Status des Erreichens des Flow-Zustands für die Aktivität durch den Benutzer, dem Fokusindex (304), dem Stressindex (306), während der Zeitdauer.

7. Verfahren nach einem der vorstehenden Ansprüche, ferner umfassend:
Bestimmen, ob der Stressindex (306) seinen vordefinierten akzeptablen Schwellenwert überschreitet;
wenn bestimmt wird, dass der Stressindex seinen vordefinierten akzeptablen Schwellenwert überschreitet, Erzeugen einer Empfehlung, eine Aufgabe durchzuführen, wobei die Aufgabe, wenn sie durch den Benutzer durchgeführt wird, ein Absenken des Stressindex ermöglicht; und
Bereitstellen einer Darstellung der Empfehlung auf einer interaktiven Benutzerschnittstelle (300).

8. Verfahren nach einem der vorstehenden Ansprüche, ferner umfassend, wenn der Benutzer den Flow-Zustand für die Aktivität erreicht hat, Erzeugen eines zweiten Steuersignals zur Steuerung mindestens eines dem Benutzer zugeordneten Kommunikationsmittels.

9. System (200) zur Überwachung des Erreichens eines Flow-Zustands für eine Aktivität durch einen Benutzer, wobei das System umfasst:
ein Blickverfolgungsmittel (202);
eine Herzfrequenzüberwachungsvorrichtung (204);
ein Verfolgungsmittel (208); und
mindestens einen Prozessor (206), der konfiguriert ist zum:
Empfangen von ersten Sensordaten von dem Blickverfolgungsmittel, und von zweiten Sensordaten von der Herzfrequenzüberwachungsvorrichtung, wobei die ersten Sensordaten und die zweiten Sensordaten erhoben werden, während der Benutzer die Aktivität ausführt;
Bestimmen einer Blickfixierungsfrequenz und eines Blickfixierungsprozentsatzes durch Verarbeiten der ersten Sensordaten, und Bestimmen einer Herzfrequenz und einer Herzfrequenzvariabilität durch Verarbeiten der zweiten Sensordaten;
Bestimmen eines Fokusindex (304) des Benutzers, basierend auf der Blickfixierungsfrequenz und dem Blickfixierungsprozentsatz;
Bestimmen eines Stressindex (306) des Benutzers, basierend auf der Herzfrequenz und der Herzfrequenzvariabilität;
Bestimmen eines Flow-Index (308) des Benutzers, basierend auf einer Differenz zwischen dem Fokusindex und dem Stressindex;
Erfassen, dass der Benutzer den Flow-Zustand für die Aktivität erreicht hat, wenn der bestimmte Flow-Index gleich oder größer als ein vordefinierter Flow-Index für die Aktivität ist;
Empfangen von dritten Sensordaten von dem Verfolgungsmittel; und
Bestimmen von mindestens einem von: einer Kopfhaltung des Benutzers, einer Haltung einer am Kopf montierten Vorrichtung, die von dem Benutzer am Kopf des Benutzers getragen wird, einer Haltung einer Benutzerinteraktionssteuerung, die von dem Benutzer gehalten oder getragen wird, durch Verarbeiten der dritten Sensordaten,
wobei das mindestens eine von: der Kopfhaltung des Benutzers, der Haltung der kopfmontierten Vorrichtung, die von dem Benutzer am Kopf des Benutzers getragen wird, der Haltung der von dem Benutzer getragenen Benutzerinteraktionssteuerung verwendet wird, wenn mindestens eines bestimmt wird von: dem Fokusindex (304), dem Stressindex (306), einem Ermüdungsindex (310).

10. System nach Anspruch 9, wobei der mindestens eine Prozessor (206) ferner konfiguriert ist zum:
Bestimmen von mindestens einem von: einer Lidschlagrate, einer Lidschlaggeschwindigkeit, einem Lidschlagmuster, durch Verarbeiten der ersten Sensordaten; und
Bestimmen eines Ermüdungsindex (310) des Benutzers, basierend mindestens auf dem mindestens einen von: der Lidschlagrate, der Lidschlaggeschwindigkeit, dem Lidschlagmuster;
wobei das Bestimmen des Flow-Index (308) des Benutzers auch auf dem Ermüdungsindex basiert.

11. System nach einem der Ansprüche 9 oder 10, wobei der mindestens eine Prozessor (206) ferner konfiguriert ist zum:
Empfangen von Kalibrierungsdaten von dem Blickverfolgungsmittel (202) und der Herzfrequenzüberwachungsvorrichtung (204), wobei die Kalibrierungsdaten während einer Kalibrierungszeitspanne erhoben werden; und
Bestimmen eines Basislinienfokusindex und eines Basislinienstressindex für den Benutzer durch Verarbeiten der Kalibrierungsdaten,
wobei das Bestimmen des Flow-Index (308) des Benutzers auch auf dem Basislinienfokusindex und dem Basislinienstressindex basiert.

12. System nach einem der Ansprüche 9 bis 11, wobei der mindestens eine Prozessor (206) ferner konfiguriert ist, um mindestens eines durchzuführen von:
Erzeugen einer Visualisierung (302, 316), die mindestens den Fokusindex (304), den Stressindex (306) und den Flow-Index (308) angibt, und Bereitstellen der Visualisierung auf einer interaktiven Benutzerschnittstelle (300);
Bestimmen des Flow-Index für eine zweite Zeitspanne, und Bereitstellen einer Darstellung (312) des Flow-Index für die zweite Zeitspanne auf einer interaktiven Benutzerschnittstelle; und
Bestimmen einer dritten Zeitspanne an einem bestimmten Tag, für die der Flow-Index höher als ein vordefinierter Wert ist, Vergleichen der dritten Zeitspanne mit einer Ziel-Flow-Zustandszeitspanne zum Erzeugen eines Vergleichsergebnisses, und Bereitstellen, auf einer interaktiven Benutzerschnittstelle, einer Darstellung (314) von mindestens einem von: der dritten Zeitspanne, dem Vergleichsergebnis.

13. System nach einem der Ansprüche 9 bis 12, wobei der mindestens eine Prozessor (206) ferner konfiguriert ist zum:
Bereitstellen, über eine interaktive Benutzerschnittstelle (300), eines Zeitgebers (318);
Empfangen einer Benutzereingabe, die eine Zeitdauer angibt, die von dem Benutzer an dem Zeitgeber eingestellt wird, wobei der Benutzer die Aktivität für die Zeitdauer ausführt;
Erzeugen eines Steuersignals für den Zeitgeber, um nach Ablauf der Zeitdauer zu klingeln; und
nach Ablauf der Zeitdauer, Erzeugen eines Flow-Zustandsberichts, der mindestens eines angibt von: dem Flow-Index (308), einem Status des Erreichens des Flow-Zustands für die Aktivität durch den Benutzer, dem Fokusindex (304), dem Stressindex (306), während der Zeitdauer.

14. System nach einem der Ansprüche 9 bis 13, wobei der mindestens eine Prozessor (206) ferner konfiguriert ist zum:
Bestimmen, ob der Stressindex (306) seinen vordefinierten akzeptablen Schwellenwert überschreitet;
wenn bestimmt wird, dass der Stressindex seinen vordefinierten akzeptablen Schwellenwert überschreitet, Erzeugen einer Empfehlung, eine Aufgabe durchzuführen, wobei die Aufgabe, wenn sie durch den Benutzer durchgeführt wird, ein Absenken des Stressindex ermöglicht; und
Bereitstellen einer Darstellung der Empfehlung auf einer interaktiven Benutzerschnittstelle (300).

## Revendications

1. Procédé mis en œuvre par ordinateur permettant de surveiller l'acquisition d'un état de flux pour une activité par un utilisateur, le procédé comprenant :
la réception de premières données de capteur à partir d'un moyen de suivi oculaire (202), et de deuxièmes données de capteur à partir d'un dispositif de surveillance de fréquence cardiaque (204), dans lequel les premières données de capteur et les deuxièmes données de capteur sont collectées lorsque l'utilisateur exerce l'activité ;
la détermination d'une fréquence de fixation d'œil et d'un pourcentage de fixation d'œil en traitant les premières données de capteur, et la détermination d'une fréquence cardiaque et d'une variabilité de fréquence cardiaque en traitant les deuxièmes données de capteur ;
la détermination d'un indice de concentration (304) de l'utilisateur, sur la base de la fréquence de fixation d'œil et du pourcentage de fixation d'œil ;
la détermination d'un indice de stress (306) de l'utilisateur, sur la base de la fréquence cardiaque et de la variabilité de fréquence cardiaque ;
la détermination d'un indice de flux (308) de l'utilisateur, sur la base d'une différence entre l'indice de concentration et l'indice de stress ;
le fait de détecter que l'utilisateur a acquis l'état de flux pour l'activité, lorsque l'indice de flux déterminé est égal ou supérieur à un indice de flux prédéfini pour l'activité ;
la réception de troisièmes données de capteur à partir d'un moyen de suivi (208) ; et
la détermination d'au moins l'une parmi : une pose de la tête de l'utilisateur, une pose d'un dispositif monté sur la tête porté par l'utilisateur sur la tête de l'utilisateur, une pose d'un dispositif de commande d'interaction utilisateur qui est tenu ou porté par l'utilisateur, en traitant les troisièmes données de capteur,
dans lequel l'au moins une parmi : la pose de la tête de l'utilisateur, la pose du dispositif monté sur la tête porté par l'utilisateur sur la tête de l'utilisateur, la pose du dispositif de commande d'interaction utilisateur porté par l'utilisateur, est utilisée pour déterminer au moins l'un parmi : l'indice de concentration (304), l'indice de stress (306), un indice de fatigue (310).

2. Procédé selon la revendication 1, comprenant en outre :
la détermination d'au moins l'un parmi : une fréquence de clignement, une vitesse de clignement, un motif de clignement, par le traitement des premières données de capteur ; et
la détermination de l'indice de fatigue (310) de l'utilisateur, sur la base au moins de l'au moins un parmi : la fréquence de clignement, la vitesse de clignement, le motif de clignement ; dans lequel la détermination de l'indice de flux (308) de l'utilisateur est également basée sur l'indice de fatigue.

3. Procédé selon l'une quelconque des revendications précédentes, comprenant en outre :
la réception de données d'étalonnage à partir du moyen de suivi oculaire (202) et du dispositif de surveillance de fréquence cardiaque (204), dans lequel les données d'étalonnage sont collectées pendant une période de temps d'étalonnage ; et
la détermination d'un indice de concentration de référence et d'un indice de stress de référence pour l'utilisateur, en traitant les données d'étalonnage, dans lequel la détermination de l'indice de flux (308) de l'utilisateur est également basée sur l'indice de concentration de référence et l'indice de stress de référence.

4. Procédé selon l'une quelconque des revendications précédentes, comprenant en outre :
la collecte de données d'état de flux d'utilisateur comprenant des indices de flux de l'utilisateur, qui sont déterminés sur une première période de temps ; et
la détermination d'un multiplicateur de pondération de concentration et d'un multiplicateur de pondération de stress pour l'indice de concentration (304) et l'indice de stress (306), respectivement, en analysant les données d'état de flux d'utilisateur pour identifier la manière dont l'indice de concentration et l'indice de stress affectent la détermination des indices de flux, dans lequel la détermination de l'indice de flux (308) de l'utilisateur est également basée sur le multiplicateur de pondération de concentration et le multiplicateur de pondération de stress.

5. Procédé selon l'une quelconque des revendications précédentes, comprenant en outre au moins l'une parmi :
la génération d'une visualisation (302, 316) indiquant au moins l'indice de concentration (304), l'indice de stress (306) et l'indice de flux (308), et la fourniture de la visualisation sur une interface utilisateur interactive (300) ;
la détermination de l'indice de flux pour une deuxième période de temps, et la fourniture d'une représentation (312) de l'indice de flux pour la deuxième période de temps sur une interface utilisateur interactive ; et
la détermination d'une troisième période de temps dans un jour donné pour lequel l'indice de flux est supérieur à une valeur prédéfinie, la comparaison de la troisième période de temps avec une période de temps d'état de flux cible pour générer un résultat de comparaison, et la fourniture, sur une interface utilisateur interactive, d'une représentation (314) d'au moins l'un parmi : la troisième période de temps, le résultat de comparaison.

6. Procédé selon l'une quelconque des revendications précédentes, comprenant en outre :
la fourniture, par l'intermédiaire d'une interface utilisateur interactive (300), d'une minuterie (318) ;
la réception d'une entrée d'utilisateur indiquant une durée de temps qui est définie par l'utilisateur sur la minuterie, dans lequel l'utilisateur exerce l'activité pendant la durée de temps ;
la génération d'un signal de commande pour que la minuterie sonne lors de l'écoulement de la durée de temps ; et
lors de l'écoulement de la durée de temps, la génération d'un rapport d'état de flux indiquant au moins l'un parmi : l'indice de flux (308), un état de l'utilisateur acquérant l'état de flux pour l'activité, l'indice de concentration (304), l'indice de stress (306), pendant la durée de temps.

7. Procédé selon l'une quelconque des revendications précédentes, comprenant en outre :
le fait de déterminer si l'indice de stress (306) dépasse son seuil acceptable prédéfini ;
lorsqu'il est déterminé que l'indice de stress dépasse son seuil acceptable prédéfini, la génération d'une recommandation pour réaliser une tâche, dans lequel la tâche, lorsqu'elle est réalisée par l'utilisateur, permet d'abaisser l'indice de stress ; et
la fourniture d'une représentation de la recommandation sur une interface utilisateur interactive (300).

8. Procédé selon l'une quelconque des revendications précédentes, comprenant en outre, si l'utilisateur a acquis l'état de flux pour l'activité, la création d'un second signal de commande pour commander au moins un moyen de communication associé à l'utilisateur.

9. Système (200) de surveillance de l'acquisition d'un état de flux pour une activité par un utilisateur, le système comprenant :
un moyen de suivi oculaire (202) ;
un dispositif de surveillance de fréquence cardiaque (204) ;
un moyen de suivi (208) ; et
au moins un processeur (206) configuré pour :
recevoir de premières données de capteur à partir du moyen de suivi oculaire, et de deuxièmes données de capteur à partir du dispositif de surveillance de fréquence cardiaque, dans lequel les premières données de capteur et les deuxièmes données de capteur sont collectées lorsque l'utilisateur exerce l'activité ;
déterminer une fréquence de fixation d'œil et un pourcentage de fixation d'œil en traitant les premières données de capteur, et déterminer une fréquence cardiaque et une variabilité de fréquence cardiaque en traitant les deuxièmes données de capteur ;
déterminer un indice de concentration (304) de l'utilisateur, sur la base de la fréquence de fixation d'œil et du pourcentage de fixation d'œil ;
déterminer un indice de stress (306) de l'utilisateur, sur la base de la fréquence cardiaque et de la variabilité de fréquence cardiaque ;
déterminer un indice de flux (308) de l'utilisateur, sur la base d'une différence entre l'indice de concentration et l'indice de stress ;
détecter que l'utilisateur a acquis l'état de flux pour l'activité, lorsque l'indice de flux déterminé est égal ou supérieur à un indice de flux prédéfini pour l'activité ;
recevoir de troisièmes données de capteur à partir du moyen de suivi ; et
déterminer au moins l'une parmi : une pose de la tête de l'utilisateur, une pose d'un dispositif monté sur la tête porté par l'utilisateur sur la tête de l'utilisateur, une pose d'un dispositif de commande d'interaction utilisateur qui est tenu ou porté par l'utilisateur, en traitant les troisièmes données de capteur,
dans lequel l'au moins une parmi : la pose de la tête de l'utilisateur, la pose du dispositif monté sur la tête porté par l'utilisateur sur la tête de l'utilisateur, la pose du dispositif de commande d'interaction utilisateur porté par l'utilisateur, est utilisée lors de la détermination d'au moins l'un parmi : l'indice de concentration (304), l'indice de stress (306), un indice de fatigue (310).

10. Système selon la revendication 9, dans lequel l'au moins un processeur (206) est configuré en outre pour :
déterminer au moins l'un parmi : une fréquence de clignement, une vitesse de clignement, un motif de clignement, en traitant les premières données de capteur ; et
déterminer un indice de fatigue (310) de l'utilisateur, sur la base au moins de l'au moins un parmi : la fréquence de clignement, la vitesse de clignement, le motif de clignement ;
dans lequel la détermination de l'indice de flux (308) de l'utilisateur est également basée sur l'indice de fatigue.

11. Système selon l'une quelconque des revendications 9 à 10, dans lequel l'au moins un processeur (206) est configuré en outre pour :
recevoir des données d'étalonnage à partir du moyen de suivi oculaire (202) et du dispositif de surveillance de fréquence cardiaque (204), dans lequel les données d'étalonnage sont collectées pendant une période de temps d'étalonnage ; et
déterminer un indice de concentration de référence et un indice de stress de référence pour l'utilisateur, en traitant les données d'étalonnage,
dans lequel la détermination de l'indice d'écoulement (308) de l'utilisateur est également basée sur l'indice de concentration de référence et l'indice de stress de référence.

12. Système selon l'une quelconque des revendications 9 à 11, dans lequel l'au moins un processeur (206) est configuré en outre pour réaliser au moins l'un parmi :
générer une visualisation (302, 316) indiquant au moins l'indice de concentration (304), l'indice de stress (306) et l'indice de flux (308), et fournissant la visualisation sur une interface utilisateur interactive (300) ;
déterminer l'indice de flux pour une deuxième période de temps, et fournissant une représentation (312) de l'indice de flux pour la deuxième période de temps sur une interface utilisateur interactive ; et
déterminer une troisième période de temps dans un jour donné pour lequel l'indice de flux est supérieur à une valeur prédéfinie, comparant la troisième période de temps avec une période de temps d'état de flux cible pour générer un résultat de comparaison, et fournissant, sur une interface utilisateur interactive, une représentation (314) d'au moins l'un parmi : la troisième période de temps, le résultat de comparaison.

13. Système selon l'une quelconque des revendications 9 à 12, dans lequel l'au moins un processeur (206) est configuré en outre pour :
fournir, par l'intermédiaire d'une interface utilisateur interactive (300), une minuterie (318) ;
recevoir une entrée d'utilisateur indiquant une durée de temps qui est définie par l'utilisateur sur la minuterie, dans lequel l'utilisateur exerce l'activité pendant la durée de temps ;
générer un signal de commande pour que la minuterie sonne lors de l'écoulement de la durée de temps ; et
lors de l'écoulement de la durée du temps, générer un rapport d'état de flux indiquant au moins l'un parmi : l'indice de flux (308), un état de l'utilisateur acquérant l'état de flux pour l'activité, l'indice de concentration (304), l'indice de stress (306), pendant la durée de temps.

14. Système selon l'une quelconque des revendications 9 à 13, dans lequel l'au moins un processeur (206) est configuré en outre pour :
déterminer si l'indice de stress (306) dépasse son seuil acceptable prédéfini ;
lorsqu'il est déterminé que l'indice de stress dépasse son seuil acceptable prédéfini, générer une recommandation pour réaliser une tâche, dans lequel la tâche, lorsqu'elle est réalisée par l'utilisateur, permet d'abaisser l'indice de stress ; et
fournir une représentation de la recommandation sur une interface utilisateur interactive (300).
